# EUROPEAN PATENT APPLICATION

(11) **EP 4 428 236 A1**
(43) Date of publication of application: **11.09.2024**
(21) Application number: 22887250.3
(22) Date of filing: 22.03.2022
(51) Int. Cl.: C12N 15/113

(54) **ANTISENSE OLIGONUCLEOTIDES**

(30) Priority: 01.11.2021 KR 20210148357
(71) Applicant: Autotelic Bio Inc., Cheongju-si, Chungcheongbuk-do 28160 (KR)
(72) Inventor: KOO, Ji Hye, Suwon-si Gyeonggi-do 16313 (KR); KWON, Hye Jeong, Seongnam-si Gyeonggi-do 13504 (KR); KIM, Ji-Hye, Gimpo-si Gyeonggi-do 10073 (KR); PARK, Jun Eui, Seongnam-si Gyeonggi-do 13623 (KR); HONG, Soo Jeong, Chungju-si Chungcheongbuk-do 27373 (KR); PARK, Chang Hee, Hwaseong-si Gyeonggi-do 18477 (KR); KIM, Tae Hun, Sejong 30043 (KR)
(74) Representative: Mewburn Ellis LLP
(86) International application number: PCT/KR2022/003884
(87) International publication number: WO 2023/075043

(57) **Abstract**

The present invention relates to novel antisense oligonucleotides comprising at least one modified nucleotide from among oligonucleotides of SEQ ID NOs: 1 to 3. The novel oligonucleotides exhibit an excellent effect of inhibiting the expression of TGF-β2 protein, an excellent effect of killing cancer cells, and improved stability in plasma. Therefore, the oligonucleotides of the present invention can be effectively used as a pharmaceutical composition for treating diseases related to TGF-β2 expression.

## Description

### Technical Field

The present disclosure relates to antisense oligonucleotides and a pharmaceutical composition including the same.

### Background Art

After information in DNA sequences of cells is transcribed into mRNA, proteins are produced through a process in which tRNA transfers corresponding amino acids based on information of mRNA from ribosomes followed by ligation thereof via peptide bonds.

If there is a single strands of DNA or RNA having a sequence complementary to the mRNA in the course of this gene expression, this complementary DNA or RNA can bind to the mRNA to form a double chain, thereby blocking protein production.

An oligonucleotide of the complementary DNA or RNA that can bind to mRNA (sense RNA), which can be transcribed and translated into a protein as described above, forming a double chain to inhibit its function is called an antisense RNA or antisense oligonucleotide.

### Disclosure of the Invention

### Technical Goals

As a result of research to develop an antisense oligonucleotide with improved ability of inhibiting TGF-β mRNA expression, the inventors of the present invention found that a novel antisense oligonucleotide in which structure of some sugars was modified among antisense oligonucleotides exhibited excellent TGF-β inhibitory effects and high stability.

Accordingly, the present disclosure is intended to provide a novel antisense oligonucleotide and a pharmaceutical composition containing the same.

### Technical Solutions

The present disclosure relates to a novel antisense oligonucleotide including at least one modified nucleoside from among an oligonucleotide of 5'-GGCGG CATGT CTATT TTGTA-3' represented by SEQ ID NO: 1; an oligonucleotide of 5'-CGGCA TGTCT ATTTT GTAAA-3' represented by SEQ ID NO: 2; or an oligonucleotide of 5'-GCGGC ATGTC TATTT TGTAA-3' represented by SEQ ID NO: 3.

Specifically, the present disclosure relates to an antisense oligonucleotide of any one of SEQ ID NOS: 1 to 3, wherein the antisense oligonucleotide has a nucleotide in which at least one nucleoside of the oligonucleotide is modified.

Herein, the nucleotide with the modified nucleoside may be a 2'-O-methoxyethyl (MOE)-modified nucleotide, a 2'-fluoro (F)-modified nucleotide, a 2'-O-methyl (O-Me)-modified nucleotide, a locked nucleic acid (LNA)-modified nucleotide, an ethylene-bridged nucleic acid (ENA)-modified nucleotide, an (R/S)-constrained ethyl (cET)-modified nucleotide, or a polyalkylene oxide (e.g., triethylene glycol (TEG))-modified nucleotide. (Hereinafter, unless otherwise specified, nucleotides with nucleosides modified as described above are referred to as "modified nucleotides")

For example, the antisense oligonucleotide according to the present disclosure may have a 2' position on a ribose pentose of a modified nucleoside in a 2'-O-methoxyethyl (MOE) form, a 2'-fluoro form, or a 2'-O-methyl form, or may be in the form of LNA or ENA in which oxygen at the 2' position on the ribose pentose of the modified nucleoside is connected to carbon at a 4' position or in the form of cET where a bridge that connects oxygen at the 2' position and carbon at the 4' position of the LNA is substituted with a methyl group.

A structure of the modified nucleoside illustrated above may be represented as follows:

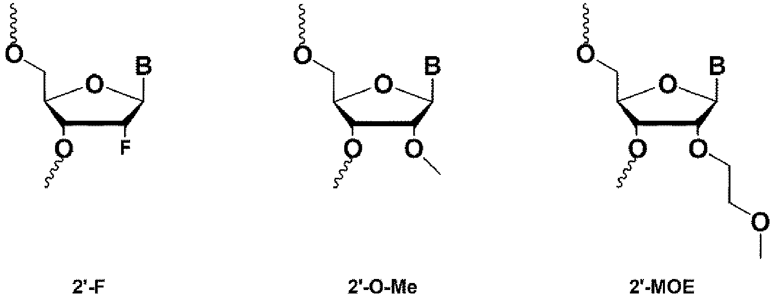

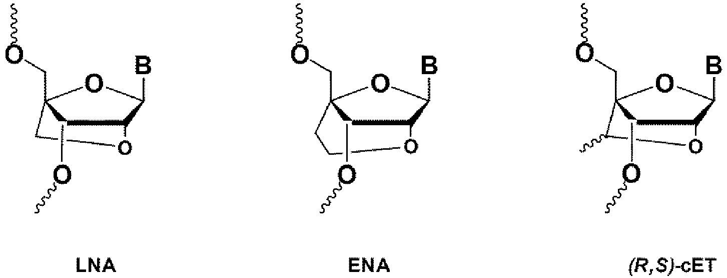

The oligonucleotide of the present disclosure includes one or more modified nucleosides at a 5'-end and/or a 3' end. For example, the oligonucleotide includes one or more modified nucleosides at the 5'-end or 3'-end of the oligonucleotide of the present disclosure. Thus, a nucleotide having at least one modified nucleoside at the 5'-end or 3'-end of the oligonucleotide of the present disclosure, i.e., a "modified nucleotide" may be included.

Specifically, the oligonucleotide according to the present disclosure is an oligonucleotide in which one or more nucleotides at the 5'-end or one or more nucleotides at the 3'-end of the oligonucleotide are modified nucleotides.

In addition, the oligonucleotide according to the present disclosure is an oligonucleotide in which one or more nucleotides at the 5'-end and one or more nucleotides at the 3'-end of the oligonucleotide are modified nucleotides.

In the present disclosure, the first to n^{th} nucleotides at the 5'-end of the oligonucleotide or the first to m^{th} nucleotides at the 3'-end of the oligonucleotide may be the modified nucleotides.

In addition, in the present disclosure, the first to n^{th} nucleotides at the 5'-end of the oligonucleotide and the first to m^{th} nucleotides at the 3'-end of the oligonucleotide may be the modified nucleotides.

Herein, n and m are each independently an integer of 1 to 10, preferably n and m are each independently an integer of 1 to 9, and more preferably n and m are each independently an integer of 1 to 7.

In addition, the oligonucleotide of the present disclosure may further include a modified nucleotide between the modified nucleosides present at the 5'-end or the 3'-end.

In other words, the oligonucleotide may further include one or more modified nucleotides between the (n+1)^{th} nucleotide at the 5'-end and the (m+1)^{th} nucleotide at the 3'- end of the oligonucleotide.

A preferred example of the oligonucleotide of the present disclosure may be any one of antisense oligonucleotides indicated by 1 to 30 in the following Table.

In Table 1 below, underlined, bold nucleotides indicate modified nucleotides.

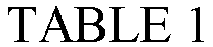

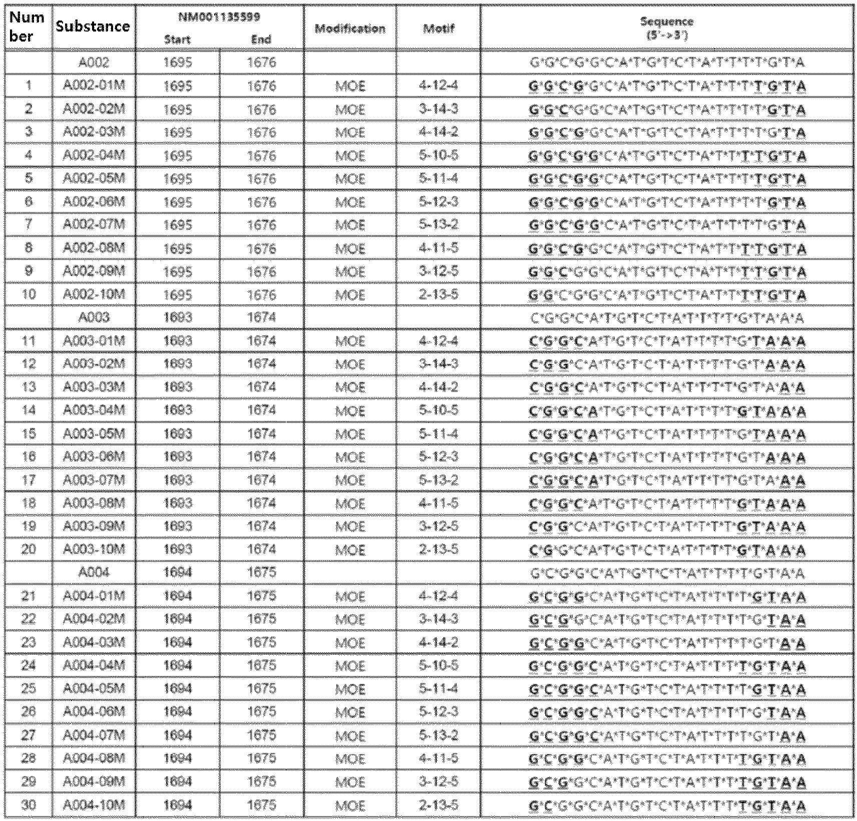

The modified nucleotide of the oligonucleotide in Table 1 (indicated in underlined, bold letters) is a 2'-O-methoxyethyl (MOE)-modified nucleotide.

Here, GenBank Access No. NM_001135599 in Table 1 is known as the sequence that expresses TGF-β2, as shown below (see SEQ ID NO: 4):

Genebank NM001135599

1 gatgttatct gctggcagca gaaggttcgc tccgagcgga gctccagaag ctcctgacaa
61 gagaaagaca gattgagata gagatagaaa gagaaagaga gaaagagaca gcagagcgag
121 agcgcaagtg aaagaggcag gggaggggga tggagaatat tagcctgacg gtctagggag
181 tcatccagga acaaactgag gggctgcccg gctgcagaca ggaggagaca gagaggatct
241 atttaggggt ggcaagtgcc tacctaccct aagcgagcaa ttccacgttg gggagaagcc
301 agcagaggtt gggaaagggt gggagtccaa gggagcccct gcgcaacccc ctcaggaata
361 aaactcccca gccagggtgt cgcaagggct gccgttgtga tccgcagggg gtgaacgcaa
421 ccgcgacggc tgatcgtctg tggctgggtt ggcgtttgga gcaagagaag gaggagcagg
481 agaaggaggg agctggaggc tggaagcgtt tgcaagcggc ggcggcagca acgtggagta
541 accaagcggg tcagcgcgcg cccgccaggg tgtaggccac ggagcgcagc tcccagagca
601 ggatccgcgc cgcctcagca gcctctgcgg cccctgcggc acccgaccga gtaccgagcg
661 ccctgcgaag cgcaccctcc tccccgcggt gcgctgggct cgcccccagc gcgcgcacac
721 gcacacacac acacacacac acacacgcac gcacacacgt gtgcgcttct ctgctccgga
781 gctgctgctg ctcctgctct cagcgccgca gtggaaggca ggaccgaacc gctccttctt
841 taaatatata aatttcagcc caggtcagcc tcggcggccc ccctcaccgc gctcccggcg
901 cccctcccgt cagttcgcca gctgccagcc ccgggacctt ttcatctctt cccttttggc
961 cggaggagcc gagttcagat ccgccactcc gcacccgaga ctgacacact gaactccact
1021 tcctcctctt aaatttattt ctacttaata gccactcgtc tctttttttc cccatctcat
1081 tgctccaaga atttttttct tcttactcgc caaagtcagg gttccctctg cccgtcccgt
1141 attaatattt ccacttttgg aactactggc cttttctttt taaaggaatt caagcaggat
1201 acgtttttct gttgggcatt gactagattg tttgcaaaag tttcgcatca aaaacaacaa
1261 caacaaaaaa ccaaacaact ctccttgatc tatactttga gaattgttga tttctttttt
1321 ttattctgac ttttaaaaac aacttttttt tccacttttt taaaaaatgc actactgtgt
1381 gctgagcgct tttctgatcc tgcatctggt cacggtcgcg ctcagcctgt ctacctgcag
1441 cacactcgat atggaccagt tcatgcgcaa gaggatcgag gcgatccgcg ggcagatcct
1501 gagcaagctg aagctcacca gtcccccaga agactatcct gagcccgagg aagtcccccc
1561 ggaggtgatt tccatctaca acagcaccag ggacttgctc caggagaagg cgagccggag
1621 ggcggccgcc tgcgagcgcg agaggagcga cgaagagtac tacgccaagg aggtttacaa
1681 aatagacatg ccgcccttct tcccctccga aactgtctgc ccagttgtta caacaccctc
1741 tggctcagtg ggcagcttgt gctccagaca gtcccaggtg ctctgtgggt accttgatgc
1801 catcccgccc actttctaca gaccctactt cagaattgtt cgatttgacg tctcagcaat
1861 ggagaagaat gcttccaatt tggtgaaagc agagttcaga gtctttcgtt tgcagaaccc
1921 aaaagccaga gtgcctgaac aacggattga gctatatcag attctcaagt ccaaagattt
1981 aacatctcca acccagcgct acatcgacag caaagttgtg aaaacaagag cagaaggcga
2041 atggctctcc ttcgatgtaa ctgatgctgt tcatgaatgg cttcaccata aagacaggaa
2101 cctgggattt aaaataagct tacactgtcc ctgctgcact tttgtaccat ctaataatta
2161 catcatccca aataaaagtg aagaactaga agcaagattt gcaggtattg atggcacctc
2221 cacatatacc agtggtgatc agaaaactat aaagtccact aggaaaaaaa acagtgggaa
2281 gaccccacat ctcctgctaa tgttattgcc ctcctacaga cttgagtcac aacagaccaa
2341 ccggcggaag aagcgtgctt tggatgcggc ctattgcttt agaaatgtgc aggataattg
2401 ctgcctacgt ccactttaca ttgatttcaa gagggatcta gggtggaaat ggatacacga
2461 acccaaaggg tacaatgcca acttctgtgc tggagcatgc ccgtatttat ggagttcaga
2521 cactcagcac agcagggtcc tgagcttata taataccata aatccagaag catctgcttc
2581 tccttgctgc gtgtcccaag atttagaacc tctaaccatt ctctactaca ttggcaaaac
2641 acccaagatt gaacagcttt ctaatatgat tgtaaagtct tgcaaatgca gctaaaattc
2701 ttggaaaagt ggcaagacca aaatgacaat gatgatgata atgatgatga cgacgacaac
2761 gatgatgctt gtaacaagaa aacataagag agccttggtt catcagtgtt aaaaaatttt
2821 tgaaaaggcg gtactagttc agacactttg gaagtttgtg ttctgtttgt taaaactggc
2881 atctgacaca aaaaaagttg aaggccttat tctacatttc acctactttg taagtgagag
2941 agacaagaag caaatttttt ttaaagaaaa aaataaacac tggaagaatt tattagtgtt
3001 aattatgtga acaacgacaa caacaacaac aacaacaaac aggaaaatcc cattaagtgg
3061 agttgctgta cgtaccgttc ctatcccgcg cctcacttga tttttctgta ttgctatgca
3121 ataggcaccc ttcccattct tactcttaga gttaacagtg agttatttat tgtgtgttac
3181 tatataatga acgtttcatt gcccttggaa aataaaacag gtgtataaag tggagaccaa
3241 atactttgcc agaaactcat ggatggctta aggaacttga actcaaacga gccagaaaaa
3301 aagaggtcat attaatggga tgaaaaccca agtgagttat tatatgaccg agaaagtctg
3361 cattaagata aagaccctga aaacacatgt tatgtatcag ctgcctaagg aagcttcttg
3421 taaggtccaa aaactaaaaa gactgttaat aaaagaaact ttcagtcaga ataagtctgt
3481 aagttttttt ttttcttttt aattgtaaat ggttctttgt cagtttagta aaccagtgaa
3541 atgttgaaat gttttgacat gtactggtca aacttcagac cttaaaatat tgctgtatag
3601 ctatgctata ggttttttcc tttgttttgg tatatgtaac catacctata ttattaaaat
3661 agatggatat agaagccagc ataattgaaa acacatctgc agatctcttt tgcaaactat
3721 taaatcaaaa cattaactac tttatgtgta atgtgtaaat ttttaccata ttttttatat
3781 tctgtaataa tgtcaactat gatttagatt gacttaaatt tgggctcttt ttaatgatca
3841 ctcacaaatg tatgtttctt ttagctggcc agtacttttg agtaaagccc ctatagtttg
3901 acttgcacta caaatgcatt ttttttttaa taacatttgc cctacttgtg ctttgtgttt
3961 ctttcattat tatgacataa gctacctggg tccacttgtc ttttcttttt tttgtttcac
4021 agaaaagatg ggttcgagtt cagtggtctt catcttccaa gcatcattac taaccaagtc
4081 agacgttaac aaatttttat gttaggaaaa ggaggaatgt tatagataca tagaaaattg
4141 aagtaaaatg ttttcatttt agcaaggatt tagggttcta actaaaactc agaatcttta
4201 ttgagttaag aaaagtttct ctaccttggt ttaatcaata tttttgtaaa atcctattgt
4261 tattacaaag aggacacttc ataggaaaca tctttttctt tagtcaggtt tttaatattc
4321 agggggaaat tgaaagatat atattttagt cgatttttca aaaggggaaa aaagtccagg
4381 tcagcataag tcattttgtg tatttcactg aagttataag gtttttataa atgttctttg
4441 aaggggaaaa ggcacaagcc aatttttcct atgatcaaaa aattctttct ttcctctgag
4501 tgagagttat ctatatctga ggctaaagtt taccttgctt taataaataa tttgccacat
4561 cattgcagaa gaggtatcct catgctgggg ttaatagaat atgtcagttt atcacttgtc
4621 gcttatttag ctttaaaata aaaattaata ggcaaagcaa tggaatattt gcagtttcac
4681 ctaaagagca gcataaggag gcgggaatcc aaagtgaagt tgtttgatat ggtctacttc
4741 ttttttggaa tttcctgacc attaattaaa gaattggatt tgcaagtttg aaaactggaa
4801 aagcaagaga tgggatgcca taatagtaaa cagcccttgt gttggatgta acccaatccc
4861 agatttgagt gtgtgttgat tatttttttg tcttccactt ttctattatg tgtaaatcac
4921 ttttatttct gcagacattt tcctctcaga taggatgaca ttttgttttg tattattttg
4981 tctttcctca tgaatgcact gataatattt taaatgctct attttaagat ctcttgaatc
5041 tgttttttt tttttaatt tgggggttct gtaaggtctt tatttcccat aagtaaatat
5101 tgccatggga ggggggtgga ggtggcaagg aaggggtgaa gtgctagtat gcaagtgggc
5161 agcaattatt tttgtgttaa tcagcagtac aatttgatcg ttggcatggt taaaaaatgg
5221 aatataagat tagctgtttt gtattttgat gaccaattac gctgtatttt aacacgatgt
5281 atgtctgttt ttgtggtgct ctagtggtaa ataaattatt tcgatgatat gtggatgtct
5341 ttttcctatc agtaccatca tcgagtctag aaaacacctg tgatgcaata agactatctc
5401 aagctggaaa agtcatacca cctttccgat tgccctctgt gctttctccc ttaaggacag
5461 tcacttcaga agtcatgctt taaagcacaa gagtcaggcc atatccatca aggatagaag
5521 aaatccctgt gccgtctttt tattccctta tttattgcta tttggtaatt gtttgagatt
5581 tagtttccat ccagcttgac tgccgaccag aaaaaatgca gagagatgtt tgcaccatgc
5641 tttggctttc tggttctatg ttctgccaac gccagggcca aaagaactgg tctagacagt
5701 atcccctgta gccccataac ttggatagtt gctgagccag ccagatataa caagagccac
5761 gtgctttctg gggttggttg tttgggatca gctacttgcc tgtcagtttc actggtacca
5821 ctgcaccaca aacaaaaaaa cccaccctat ttcctccaat ttttttggct gctacctaca
5881 agaccagact cctcaaacga gttgccaatc tcttaataaa taggattaat aaaaaaagta
5941 attgtgactc aa

For example, an A002 oligonucleotide in Table 1 represents an antisense oligonucleotide that binds complementarily to SEQ ID NO: 1695 to 1676 in the NM_001135599 sequence, and an A002-01M oligonucleotide represents a nucleotide in which 4 nucleotides at 5' position and 4 nucleotides at 3' position among 20 nucleotides in A002 oligonucleotides are modified (preferably, a 2'-O-methoxyethyl (MOE)-modified nucleotide).

In the oligonucleotide of the present disclosure, the bond between nucleosides, regardless of whether the nucleoside is modified, may be either phosphodiester or phosphothioate.

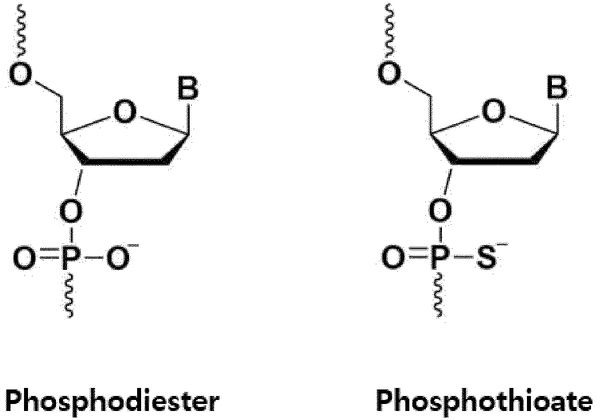

The * mark between nucleotides in the oligonucleotides in Table 1 above indicates phosphothioate.

The oligonucleotides of the present disclosure may form salts with cations. Therefore, "oligonucleotide" as used herein should be understood as a concept that also includes pharmaceutically acceptable salts of oligonucleotides.

Modification of nucleosides as described above in the present disclosure may be prepared via organochemical synthesis methods known in the art to which the present disclosure pertains.

The present disclosure also relates to a pharmaceutical composition including the antisense oligonucleotide modified as described above.

For example, the present disclosure relates to a pharmaceutical composition which includes the antisense oligonucleotide modified as described above and is to prevent or treat diseases (e.g., malignant tumors, benign tumors, immune diseases, fibrosis, or ophthalmic diseases) related to overexpression of TGF-β2 proteins.

### Advantageous Effects

When a solid tumor cell line was treated with an oligonucleotide of the present disclosure, it was found that more superior effects of inhibiting expression of TGF-β2 proteins and outstanding cancer cell killing effects were derived, and a stability in plasma was improved as well.

Therefore, the oligonucleotide of the present disclosure may be useful as a pharmaceutical composition for treating diseases related to expression of TGF-β, such as malignant tumors, benign tumors, immune diseases, fibrosis, and ophthalmic diseases.

### Brief Description of Drawings

FIG. 1 is a graph showing changes in expression of TGF-β2 mRNA by treating A549 with 20 nM ASO of the present disclosure.
FIGS. 2 and 3 are graphs showing changes in expression of TGF-β2 mRNA by treating A549 and PANC-1 with 1 µM ASO of the present disclosure.
FIGS. 4 to 6 shows uptake degrees of ASO of the present disclosure presented in graphs for each of A549, PANC-1, and A2058 cell lines.
FIG. 7 shows a result of an experiment to determine whether ASO of the present disclosure has a stability improvement effect.
FIG. 8 is a graph showing a TGF-β2 mRNA level and a protein expression level after treatment of ASO of the present disclosure by concentration for an A549 cell line.
FIGS. 9 to 11 show TGF-β2 mRNA levels upon treatment of ASO of the present disclosure by concentration for each of A549, PANC-1, and A2058 cell lines.
FIGS. 12 to 18 show TGF-β2 mRNA levels upon treatment of ASO of the present disclosure for cell lines from various carcinomas.
FIGS. 19 to 21 show tumor growth curves upon administration of ASO of the present disclosure into xenograft mice transplanted with an A2058 cell line.

### Best Mode for Carrying Out the Invention

When a solid tumor cell line was treated with an oligonucleotide of the present disclosure, it was found that more superior effects of inhibiting expression of TGF-β2 mRNA and excellent anticancer effects to suppress growth of cancer cells were derived, and a stability in plasma was improved as well.

Hereinafter, the present disclosure will be described in detail based on Examples. However, the following Examples are merely for illustrating the present disclosure, and the spirit or scope of the present disclosure is not limited thereby.

### 1. Experimental Methods (Common)

### A. Cell culture

As shown in Table 2 below, according to cell types, media such as RPMI 1640 (10% FBS, 1% antibiotic-antimycotic), DMEM (10% FBS, 1% antibiotic-antimycotic), or McCoy's 5A (10% FBS, 1% antibiotic-antimycotic) were used, and cells were cultured at 37°C in the presence of 5.0% CO₂.

**TABLE 2**

| **Tissue** | **Cell line** | **Diagnosis name** | **Composition of culture media** |
|---|---|---|---|
| **Lung** | A549 | **Carcinoma** | RPMI1640+10%FBS +1% Antibiotics |
| **Pancreas** | Panc-1 | **Epithelioid Carcinoma** | DMEM+10%FBS+1% Antibiotics |
| **Pancreas** | Capan-2 | **Adenocarcinoma** | RPMI1640+10%FBS +1% Antibiotics |
| **Pancreas** | MIA PaCa-2 | **Carcinoma** | DMEM+10%FBS+1% Antibiotics |
| **Pancreas** | BxPc-3 | **Adenocarcinoma** | RPMI1640+10%FBS+1% Antibiotics |
| **Breast** | MDA-MB-231 | **Adenocarcinoma** | RPMI1640+10%FBS+1% Antibiotics |
| **Biliary tract** | SNU-1079 | **binary tract cancer, Intrahepatic duct** | RPM11640+10%FBS+1% Antibiotics |
| **Skin** | A2058 | **Melanoma** | DMEM+10%FBS+1% Antibiotics |
| **Brain** | U-251 MG | **malignant glioblastoma** | MEM+10%FBS+1% Antibiotics |
| **Brain** | U-87 MG | **Likely Glioblastoma** | MEM+10%FBS+1% Antibiotics |

### B. Transfection with transfection reagent (Lipofectamine RNAiMAX)

Depending on the characteristics of cells and a plate size, an appropriate number of cells were seeded, and the medium was replaced after 24 hours of culture (10% FBS, without antibiotics). ASO was added in FBS-free medium in accordance with a concentration, and transfection reagents were also added in the FBS-free medium.

A mixture including ASO and a mixture including the transfection reagent were mixed in 1:1 and reacted at room temperature for 5 minutes. The mixture was dispensed into medium-replaced cell lines and cultured in an incubator (37°C, 5% CO₂) for 24 or 48 hours, depending on the purpose of the test.

Cell seeding and transfection reagents are as shown in Table 3 below:

**TABLE 3**

| Plate | Cell count | Lipofectamine RNAiMAX | Overall volume |
|---|---|---|---|
| 6 wells | 200,000-300,000 | 7.5 µl | 2 ml |
| 96 wells | 5,000-10,000 | 0.3 µl | 100 µl |

### C. Transfection without transfection reagents

Depending on the characteristics of cells and a plate size, an appropriate number of cells were seeded and cultured for 24 hours.

ASO was added in a medium including 10% FBS in accordance with a concentration.

The mixture including ASO was dispensed into the cell-seeded plates and then cultured in an incubator (37°C, 5% CO₂) for 24 or 48 hours, depending on the purpose of the test.

### 2. Quantitative analysis of TGF-β2 mRNA

### A. Extraction and quantitative analysis of RNA

After 24 or 48 hours of transfection, all culture solutions were removed, RNA was extracted using an RNA preparation kit (Rneasy Plus Mini kit, Qiagen, Cat# 74136), and then RNA was quantified by an absorbance measurement method using a micro-volume plate (Take 3, Biotek) and a multi-plate reader (Synergy H1, Biotek).

### B. Synthesis of cDNA

cDNA was synthesized with 2 µg of the extracted RNA using a cDNA Synthesis kit (RevertAid First Strand cDNA Synthesis kit, Thermo Sceientific, K1622). Synthesis was carried out under synthesis conditions according to the manufacturer's instructions, and after completion of PCR, the cDNA was diluted with distilled water (DW) to be 20 ng/µl.

Real-time PCR was performed for the quantitative analysis of TGF-β2 mRNA. Human SRSF9 was used as the reference gene.

The mixture was prepared in a composition and conditions as shown in Tables 4 to 6 below, and PCR was carried out.

**TABLE 4 - Composition of PCR mixture**

| | **Volume per one sample (ul)** |
|---|---|
| DW (ul) | 2 |
| cDNA (20 ng/ul) | 2.5 |
| Primer (F+R, 10 pmole/ul) (ul) | 0.5 |
| SYBR Green (ul) | 5 |
| Total volume (ul) | 10 |

**TABLE 5 - Primer Information**

| | | |
|---|---|---|
| Human SRSF9 | F | TGTGCAGAAGGATGGAGT |
| | R | CTGGTGCTTCTCTCAGGATA |
| Human TGF-β2 | F | CAGCACACTCGATATGGACCA |
| | R | CCTCGGGCTCAGGATAGTCT |

**TABLE 6 - PCR Conditions**

| Stage | Step | Temperature (°C) | Time | Cycle | Data collection |
|---|---|---|---|---|---|
| Hold stage | Step 1 | 50 | 2 min | 1 | |
| | Step 2 | 96 | 10 min | | |
| PCR stage | Step 1 | 96 | 15 sec | 40 | |
| | Step 2 | 60 | 1 min | | Data collection |
| Melt Curve stage | Step 1 | 96 | 15 sec | 1 | |
| | Step 2 | 60 | 1 min | | Data collection |
| | Step 3 (Dissociation) | 96 | 15 sec | | |

### 3. Quantitative analysis on expression of TGF-β2 protein (ELISA)

An appropriate number of cells were seeded, and ASO was transfected after 24 hours.

48 hours after transfection, the supernatant was collected and centrifuged (13,000 rpm, 1 minute).

Only the supernatant was collected to be subjected to quantitative analysis according to the manufacturer's instructions using a TGF-β2 ELISA kit (R&D).

### 4. Preparation of ASO substances

The ASO substance in Table 1 above was prepared by known methods that are commonly used (e.g., referring to a method described in the paper S. L. Beaucage and R. P. Iyer, Tetrahedron, 1993, 49, 6123; or S. L. Beaucage and R. P. Iyer, Tetrahedron, 1992, 48, 2223):

### Experimental Example 5. Results of treating A549 cell lines with 20 nM ASO

After treating the cell line A549 with ASO at a concentration of 20 nM in the presence of the transfection reagent, the changes in the mRNA level of TGF-β2 were compared by each candidate substance.

The culture medium for cell line A549 (lung carcinoma) was RPMI1640+10%FBS+1% antibiotics.

2×10⁵ cells/2 ml cells per well were seeded on a 6-well plate.

Transfection: After 24 hours of cell seeding, the medium was replaced (after removal of the existing culture solution, 1.8 ml of media including 10% FBS without antibiotics was added), the ASO to be tested was added in the plain media to be 20 times the treatment concentration (400 nM), and the transfection reagent (Lipofectamine RNAiMAX) was also added in the plain media to reach a concentration of 7.5 ul/100 ul. The mixture including pre-diluted ASO and the mixture including the transfection reagent were mixed in 1:1 and reacted at room temperature for 5 minutes (here, the concentration of ASO is 200 nM). To reach a final concentration for the mixture of the reacted ASO and transfection reagent to 20 nM, the mixture was dispensed by 200 ul to the medium-replaced cell lines, followed by culture for 24 hours (CO₂ incubator (37°C, 5.0% CO₂)).

Quantitative analysis of TGF-β2 mRNA:
- RNA extraction and quantification: After 24 hours of transfection, all culture solutions were removed, and then RNA was extracted using an RNA preparation kit (Rneasy Plus Mini Kit, Qiagen, Cat# 74136). After RNA extraction, RNA was quantified at OD_{260 nm} by an absorbance measurement method using a micro-volume plate (Take 3, Biotek) and a multi-plate reader (Synergy H1, Biotek).
- cDNA synthesis: cDNA was synthesized with 2 ug of the extracted RNA using a cDNA synthesis kit (RevertAid First Strand cDNA Synthesis Kit, Thermo Scientific, K1622). The synthesis conditions were followed by the manufacturer's instruction, and the cDNA was diluted with distilled water (DW) to be 20 ng/ul after completion of PCR.
- Running of real-time PCR for quantitative analysis of TGF-β2 mRNA: Using human SRSF9 as the reference gene, a PCR mixture was prepared in the same composition and conditions as in Tables 4-6 above, and PCR was performed.
- The results were identified by calculating an mRNA expression inhibition rate (%) of human TGF-β2 with a relative ratio to untreated cells (cell lines solely treated with the transfection reagent without ASO).

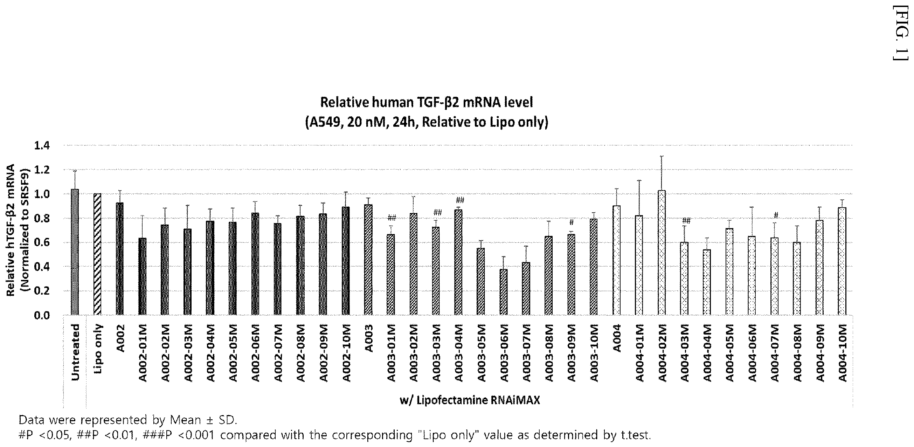

FIG. 1 shows identification of changes in human TGF-β2 mRNA levels upon treatment of 20 nM ASO (along with the transfection reagent) to A549.

The result of the experiment is as shown in Table 7 below:

**TABLE 7**

| **ASO Modification** | | **Motif** | **Concentration** | **Inhibition %** |
|---|---|---|---|---|
| A002 | - | - | 20nM | 7.6 |
| A002-01M | MOE | 4-12-4 | 20nM | 36.7 |
| A002-02M | MOE | 3-14-3 | 20nM | 25.9 |
| A002-03M | MOE | 4-14-2 | 20nM | 29.2 |
| A002-04M | MOE | 5-10-5 | 20nM | 22.6 |
| A002-05M | MOE | 5-11-4 | 20nM | 23.3 |
| A002-06M | MOE | 5-12-3 | 20nM | 15.7 |
| A002-07M | MOE | 5-13-2 | 20nM | 24.5 |
| A002-08M | MOE | 4-11-5 | 20nM | 18.4 |
| A002-09M | MOE | 3-12-5 | 20nM | 16.4 |
| A002-10M | MOE | 2-13-5 | 20nM | 11.0 |
| A003 | - | - | 20nM | 8.9 |
| A003-01M | MOE | 4-12-4 | 20nM | 33.7 |
| A003-02M | MOE | 3-14-3 | 20nM | 16.1 |
| A003-03M | MOE | 4-14-2 | 20nM | 27.7 |
| A003-04M | MOE | 5-10-5 | 20nM | 13.3 |
| A003-05M | MOE | 5-11-4 | 20nM | 45.2 |
| A003-06M | MOE | 5-12-3 | 20nM | 62.1 |
| A003-07M | MOE | 5-13-2 | 20nM | 57.0 |
| A003-08M | MOE | 4-11-5 | 20nM | 35.2 |
| A003-09M | MOE | 3-12-5 | 20nM | 33.8 |
| A003-10M | MOE | 2-13-5 | 20nM | 20.9 |
| A004 | - | - | 20nM | 9.8 |
| A004-01M | MOE | 4-12-4 | 20nM | 18.0 |
| A004-02M | MOE | 3-14-3 | 20nM | -2.8 |
| A004-03M | MOE | 4-14-2 | 20nM | 40.2 |
| A004-04M | MOE | 5-10-5 | 20nM | 46.4 |
| A004-05M | MOE | 5-11-4 | 20nM | 28.7 |
| A004-06M | MOE | 5-12-3 | 20nM | 35.3 |
| A004-07M | MOE | 5-13-2 | 20nM | 36.2 |
| A004-08M | MOE | 4-11-5 | 20nM | 40.1 |
| A004-09M | MOE | 3-12-5 | 20nM | 21.7 |
| A004-10M | MOE | 2-13-5 | 20nM | 11.2 |

### Experimental Example 6. Identification of changes in human TGF-β2 mRNA levels after treating A549 and PANC-1 cell lines with 1 uM ASO (free uptake environment)

For A549 and PANC-1 cell lines, each ASO was treated at a concentration of 1 µM in the absence of transfection reagents (free uptake environment), and changes in mRNA levels of TGF-β2 after 24 hours was compared.

### Experimental Method:

The culture medium RPMI 1640+10% FBS+1% antibiotics was used for cell line A549 (lung carcinoma), and the culture medium DMEM+10% FBS+1% antibiotics was used for cell line Panc-1 (pancreas epithelioid carcinoma).

(2-2.5)×10⁵ cells/1.8 ml of cells per well were seeded in a 6-well plate.

Transfection:
- Culture was performed for 24 hours after cell seeding.
- ASO was added in culture medium including 10% FBS to reach a concentration (10 uM) 10 times higher than that to be finally treated.
- The mixture including ASO was dispensed by 200µl to reach a final concentration of 1 uM on a cell-seeded plate, followed by culture for 24 hours (CO₂ incubator (37°C, 5.0% CO₂)).

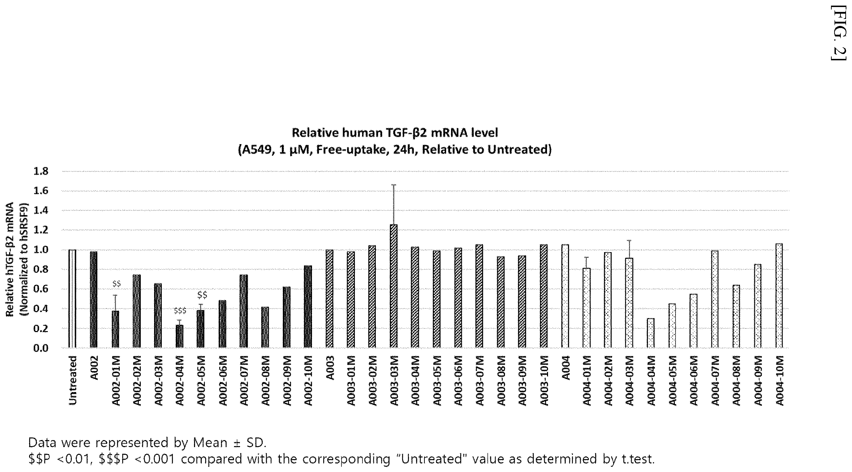

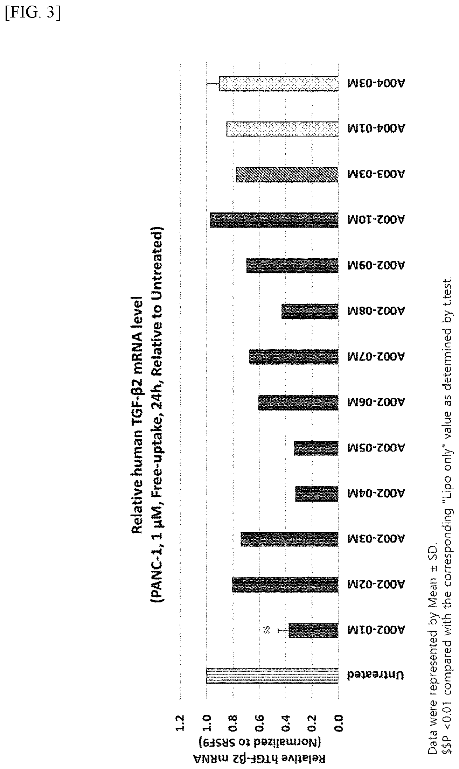

Quantitative analysis of TGF-β2 mRNA:
- RNA extraction and quantification: After 24 hours of transfection, all culture solutions were removed, and RNA was extracted using an RNA preparation kit (Rneasy Plus Mini Kit, Qiagen, Cat# 74136). After RNA extraction, the concentration was calculated at OD₂₆₀ₙₘ by an absorbance measurement method using a micro-volume plate (Take 3, Biotek) and a multi-plate reader (Synergy H1, Biotek).
- cDNA synthesis: cDNA was synthesized with 2 µg of the extracted RNA using a cDNA synthesis kit (RevertAid First Strand cDNA Synthesis Kit, Thermo Scientific, K1622). The synthesized cDNA was diluted with distilled water (DW) to be 20 ng/ul.
- Running of real-time PCR for quantitative analysis of TGF-β2 mRNA: human SRSF9 was used as the reference gene, and then the PCR mixture was prepared in the same composition and conditions as in Tables 4-6 to perform PCR.
- The results were checked by calculating an mRNA expression inhibition rate (%) of human TGF-β2 with a relative ratio to untreated cells (cell lines solely treated with transfection reagents without ASO), wherein the results for the A549 cell line are shown in Table 8 and FIG. 2 below, and the results for the PANC-1 cell line are shown in Table 9 and FIG. 3 below.

**TABLE 8**

| **ASO Modification** | | **Motif** | **Inhibition %** |
|---|---|---|---|
| A002 | - | - | 2 |
| A002-01M | MOE | 4-12-4 | 63 |
| A002-02M | MOE | 3-14-3 | 26 |
| A002-03M | MOE | 4-14-2 | 35 |
| A002-04M | MOE | 5-10-5 | 76 |
| A002-05M | MOE | 5-11-4 | 62 |
| A002-06M | MOE | 5-12-3 | 52 |
| A002-07M | MOE | 5-13-2 | 26 |
| A002-08M | MOE | 4-11-5 | 58 |
| A002-09M | MOE | 3-12-5 | 38 |
| A002-10M | MOE | 2-13-5 | 16 |
| A003 | - | - | 0 |
| A003-01M | MOE | 4-12-4 | 2 |
| A003-02M | MOE | 3-14-3 | -4 |
| A003-03M | MOE | 4-14-2 | -25 |
| A003-04M | MOE | 5-10-5 | -3 |
| A003-05M | MOE | 5-11-4 | 1 |
| A003-06M | MOE | 5-12-3 | -2 |
| A003-07M | MOE | 5-13-2 | -5 |
| A003-08M | MOE | 4-11-5 | 7 |
| A003-09M | MOE | 3-12-5 | 6 |
| A003-10M | MOE | 2-13-5 | -5 |
| A004 | - | - | -5 |
| A004-01M | MOE | 4-12-4 | 19 |
| A004-02M | MOE | 3-14-3 | 3 |
| A004-03M | MOE | 4-14-2 | 9 |
| A004-04M | MOE | 5-10-5 | 70 |
| A004-05M | MOE | 5-11-4 | 55 |
| A004-06M | MOE | 5-12-3 | 45 |
| A004-07M | MOE | 5-13-2 | 1 |
| A004-08M | MOE | 4-11-5 | 36 |
| A004-09M | MOE | 3-12-5 | 15 |
| A004-10M | MOE | 2-13-5 | -6 |

**TABLE 9**

| **ASO Modification** | | **Motif** | **% inhibition** |
|---|---|---|---|
| A002-01M | MOE | 4-12-4 | 62 |
| A002-02M | MOE | 3-14-3 | 19.7 |
| A002-03M | MOE | 4-14-2 | 26.5 |
| A002-04M | MOE | 5-10-5 | 67.5 |
| A002-05M | MOE | 5-11-4 | 66.5 |
| A002-06M | MOE | 5-12-3 | 39.7 |
| A002-07M | MOE | 5-13-2 | 32.7 |
| A002-08M | MOE | 4-11-5 | 57 |
| A002-09M | MOE | 3-12-5 | 30.4 |
| A002-10M | MOE | 2-13-5 | 2.7 |
| A003-03M | MOE | 4-14-2 | 22.3 |
| A004-01M | MOE | 4-12-4 | 17.5 |
| A004-03M | MOE | 4-14-2 | 8.9 |

### Experimental Example 7: Comparison of free uptakes in the A549 cell line using 5'-fluorescein amidite (FAM) ASO

Free uptakes (absorption under transfection reagent-free environment) were compared in A549, PANC-1, and A2058 cell lines using 5'-fluorescein amidite (FAM)-labeled ASO.

### Experimental Method:

Culture medium RPMI1640+10%FBS+1% antibiotics was used for cell line A549 (lung carcinoma), and culture medium DMEM+10%FBS+1% antibiotics was used for cell lines PANC-1 (pancreas epithelioid carcinoma) and A2058 (melanoma).

### Cell Seeding:

4×10⁵ cells/500 µl of cells per well were seeded in a 4-chamber slide.

### Transfection:

The ASO to be tested was dispensed into each well by 200 µl to reach a concentration of 1 µM in DMEM medium including 10% FBS and 1% antibiotics, followed by culture for 24 hours (CO₂ incubator 37°C, 5.0% CO₂).

### FACS analysis

Cells were detached by treating trypsin-EDTA and washed with PBS. After the cells were suspended with PBS, fluorescence-activated cell sorter (FACS) analysis was performed. Mean fluorescence intensity (MFI) was checked, and the relative content of the FAM-labeled ASO of each cell was compared.

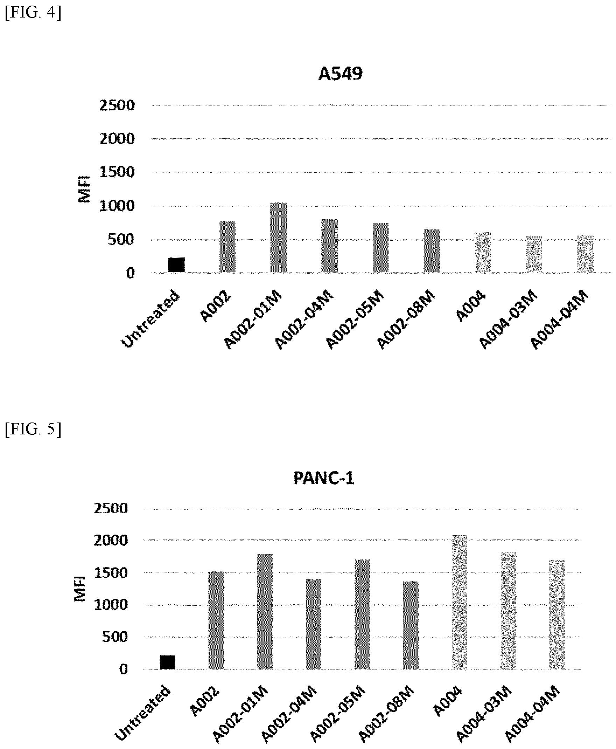

The results of MFI measurements for the A549 cell line, the PAC-1 cell line, and the A2058 cell line are shown in FIGS. 4, 5, and 6, respectively.

As a result of the experiment, it was found that all ASOs used in the test were properly absorbed into the cells.

### Experimental Example 8: Measurement of stability after spiking ASO into human plasma

ASO was spiked into human plasma and cultured for 7 days, and the amount of ASO remaining was analyzed.

### Experimental Method:

Spiking was performed to make ASO in the human plasma reach a final concentration of 5 uM and incubated for 7 days in a 37°C incubator with rotation at 40 rpm. Plasma was sampled at different times (day 0, 4 hours, day 1, day 2, and day 7), and the amount of residual ASO was analyzed by HPLC. A proportion of the remaining ASO amount was calculated based on the amount of ASO detected at hour 0.

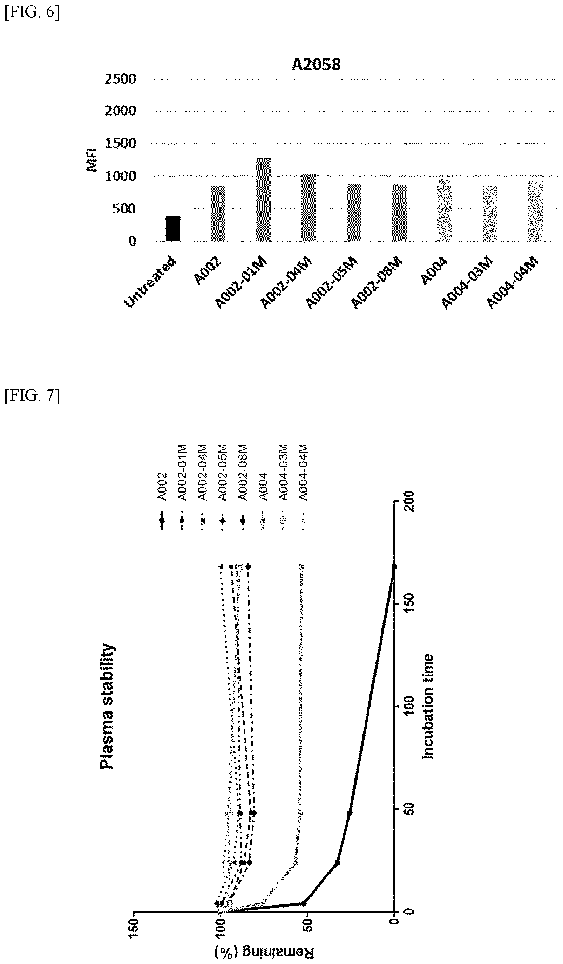

The result of the experiment is shown in FIG. 7.

The ASO substance according to the present disclosure had significantly increased stability compared to naked ASO (A002 and A004) and was found to remain in the plasma by more than 80% even after 7 days.

### Experimental Example 9: Identification of changes in levels of mRNA and protein release after treatment of ASO by concentration in the presence of the transfection reagent for A549 cell lines (5-160 nM)

After treating A549 cell lines with ASO by concentration (0 nM, 5 nM, 20 nM, 80 nM) in the presence of the transfection reagents, changes in mRNA levels and protein release levels of TGF-β2 were checked.

### Experimental Method:

The culture medium RPMI1640+10% FBS+1% antibiotics was used for cell line A549 (lung carcinoma).

2×10⁵ cells/2 ml cells per well were seeded in a 6-well plate.

### Transfection:

- 24 hours after cell seeding, the medium was replaced (after removal of the existing culture solution, 1.8 ml of medium including 10% FBS without antibiotics was added). The ASO to be tested was diluted in plain media to be 20 times the concentration to be treated.
- The transfection reagent (Lipofectamine RNAiMAX) was also diluted with plain media to reach a concentration of 7.5 µl/100 µl.
- A mixture including pre-diluted ASO and a mixture including the transfection reagent were mixed in a volume ratio of 1:1 and reacted at room temperature for 5 minutes.
- To reach the preset final concentration, a mixture of the reacted ASO and transfection reagent was dispensed by 200 ul into the medium-replaced cell line, followed by culture for 24 hours. (CO₂ incubator (37°C, 5.0% CO₂))
- The medium was replaced with 1 ml of medium which includes 1% FBS without antibiotics, followed by culture for 48 hours. (CO₂ incubator (37°C, 5.0% CO₂))

### Quantitative analysis of TGF-02 mRNA:

- RNA extraction and quantification: After 72 hours of transfection, all culture solutions were removed, and RNA was extracted using an RNA preparation kit (Rneasy Plus Mini Kit, Qiagen, Cat# 74136). After RNA extraction, the concentration was calculated at OD_{260 nm} by an absorbance measurement method using a micro-volume plate (Take 3, Biotek) and a multi-plate reader (Synergy H1, Biotek).
- cDNA synthesis: cDNA was synthesized with 2 µg of the extracted RNA using a cDNA synthesis kit (RevertAid First Strand cDNA Synthesis Kit, Thermo Scientific, K1622). The synthesized cDNA was diluted to be 20 ng/µl using distilled water (DW).
- Running of real-time PCR for TGF-β2 mRNA quantitative analysis: Human SRSF9 was used as the reference gene. The PCR mixture was prepared in the same composition and conditions as shown in Tables 4 to 6 above, and PCR was carried out.

### Quantitative analysis on TGF-β2 protein expression (ELISA)

- Separately collected culture solution was centrifuged, only the supernatant was collected, and quantitative analysis was conducted according to the manufacturer's instruction using a TGF-β2 ELISA kit (R&D).

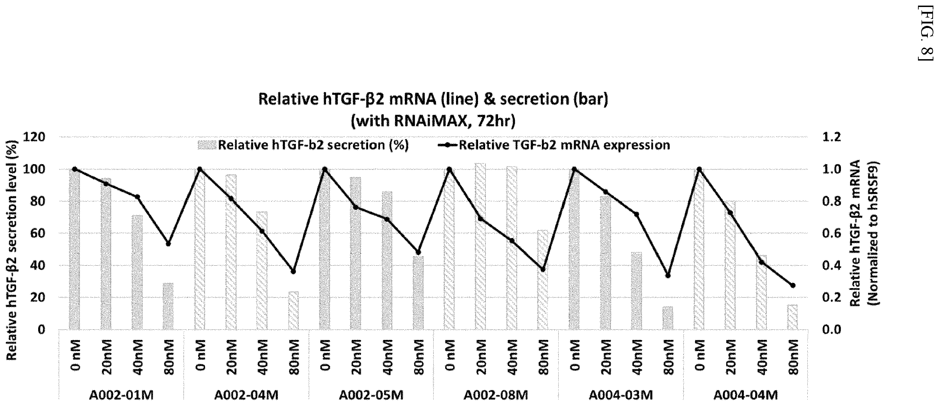

The result of the experiment is shown in FIG. 8. As a result of the experiment, it was determined that ASO according to the present disclosure decreases human TGF β2 mRNA levels and secretion levels in a concentration-dependent manner.

### Experimental Example 10: Identification of human TGF-β2 mRNA inhibition after treatment of ASO by concentration in absence of transfection reagent (free uptake condition)

After treating ASO to A549, PANC-1, and A2058 cell lines by concentration (3.9 nM-4000 nM) under free uptake conditions without the transfection reagents, changes in mRNA levels of human TGF-β2 compared to untreated cells were checked.

### Experimental Method:

The culture medium RPMI1640+10% FBS+1% antibiotics was used for cell line A549 (lung carcinoma), and the culture medium DMEM+10% FBS+1% antibiotics was used for cell lines PANC-1 (pancreas epithelioid carcinoma) and A2058 (melanoma).

(2~2.5)×10⁵ cells/2 ml of cells per well were seeded in a 6-well plate.

### Transfection:

- 24 hours after cell seeding, the medium was replaced (after removal of the existing culture solution, 1.8 ml of medium including 10% FBS without antibiotics was added). The ASO to be tested was diluted in plain media to become 10 times the concentration to be treated.
- To reach the preset final concentration, the mixture including pre-diluted ASO was dispensed by 200 µl to the medium-replaced cell line, followed by culture for 48 hours. (CO₂ incubator (37°C, 5.0% CO₂))

### Quantitative analysis of TGF-β2 mRNA:

- RNA extraction and quantification: After 48 hours of transfection, all culture solutions were removed, and RNA was extracted using an RNA preparation kit (Rneasy Plus Mini Kit, Qiagen, Cat# 74136). After RNA extraction, the concentration was calculated at OD_{260 nm} by an absorbance measurement method using a micro-volume plate (Take 3, Biotek) and a multi-plate reader (Synergy H1, Biotek).
- cDNA synthesis: cDNA was synthesized with 2 µg of the extracted RNA using a cDNA synthesis kit (RevertAid First Strand cDNA Synthesis Kit, Thermo Scientific, K1622). The synthesized cDNA was diluted to be 20 ng/µl using distilled water (DW).
- Running of real-time PCR for TGF-β2 mRNA quantitative analysis: Human SRSF9 was used as the reference gene. The PCR mixture was prepared in the same composition and conditions as shown in Tables 4 to 6 above, and PCR was carried out.

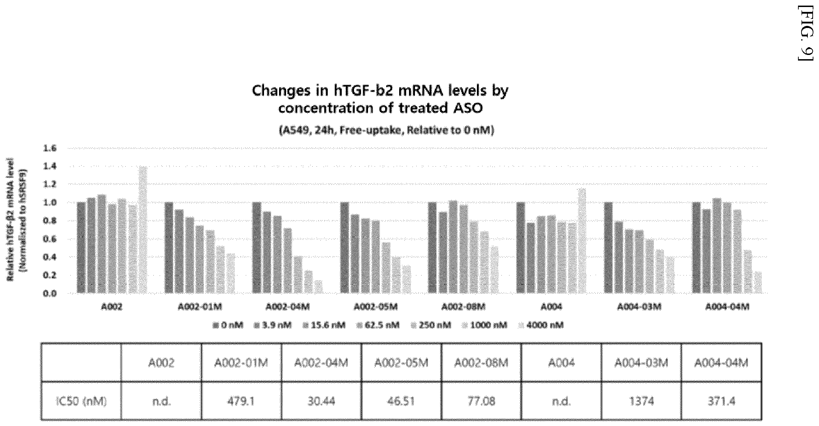

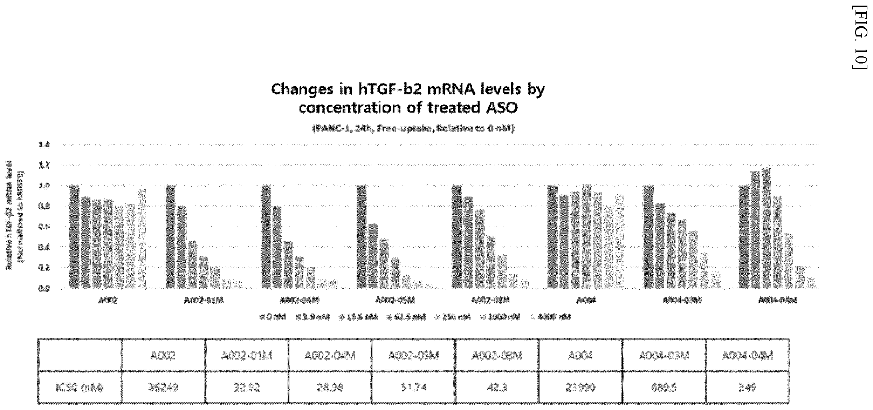

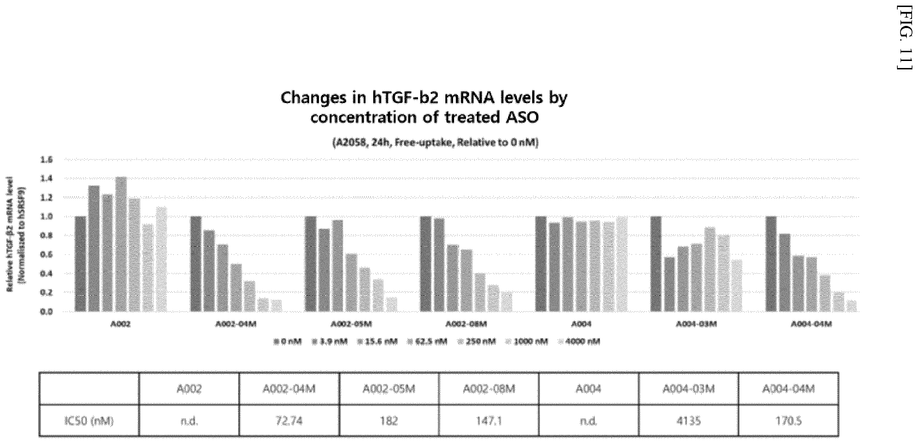

The result of the experiment for the A549 cell line, the PAC-1 cell line, and the A2058 cell line are shown in FIGS. 9, 10, and 11, respectively.

As a result of the experiment, A002-04M, A002-05M, and A002-08M had particularly superior effects of inhibiting TGF-β2 mRNA for the A549 cell line, A002-01M, A002-04M, A002-05M, and A002-08M had particularly excellent effects of inhibiting TGF-β2 mRNA for the PANC-1 cell line, and A002-04M, A002-05M, A002-08M, and A004-04M had particularly superior effects of inhibiting TGF-β2 mRNA for the A2058 cell line.

### Experimental Example 11: Identification of changes in human TGF-β2 mRNA levels after treatment of ASO in the absence of the transfection reagent (free uptake condition) for cell lines from various carcinomas

Cell lines from various carcinomas were treated with ASO, and a reduction in TGF-β2 mRNA levels was identified after 24 or 72 hours.

### Experimental Method:

The cell line of an experimental subject and medium used are the same as shown in Table 2 above.

(2∼2.5)×10⁵ cells/2 ml of cells per well were seeded in a 6-well plate.

### Transfection:

- 24 hours after cell seeding, 1.8 ml of medium including 1% FBS was added.
- The ASO to be tested was diluted in plain media to be 10 times the concentration to be treated.
- To reach the preset final concentration, the mixture including pre-diluted ASO was dispensed by 200 µl to the medium-replaced cell line, followed by culture 24 or 72 hours. (CO₂ incubator (37°C, 5.0% CO₂))

### Quantitative analysis of TGF-02 mRNA:

- RNA extraction and quantification: 24 hours after transfection, all culture solutions were removed, and RNA was extracted using an RNA preparation kit (Rneasy Plus Mini Kit, Qiagen, Cat# 74136). After RNA extraction, the concentration was calculated at OD_{260 nm} by an absorbance measurement method using a micro-volume plate (Take 3, Biotek) and a multi-plate reader (Synergy H1, Biotek).
- cDNA synthesis: cDNA was synthesized with 2 µg of the extracted RNA using a cDNA synthesis kit (RevertAid First Strand cDNA Synthesis Kit, Thermo Scientific, K1622). The synthesized cDNA was diluted to be 20 ng/µl using distilled water (DW).
- Running of real-time PCR for TGF-β2 mRNA quantitative analysis: Human SRSF9 was used as the reference gene. The PCR mixture was prepared in the same composition and conditions as shown in Tables 4 to 6 above, and PCR was carried out.

The mRNA inhibition rate (%) of human TGF-β2 was calculated with a relative ratio to untreated cells (cell lines solely treated with the transfection reagents without ASO).

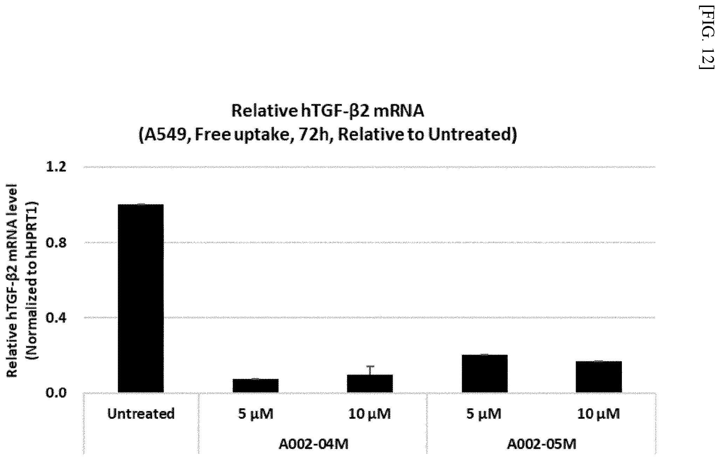

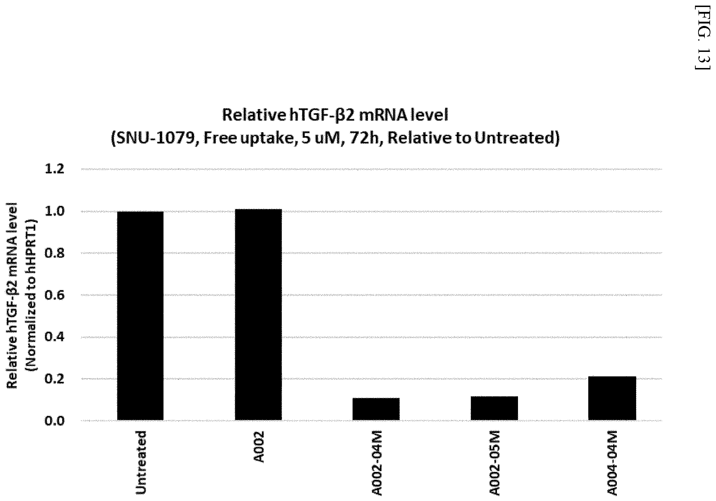

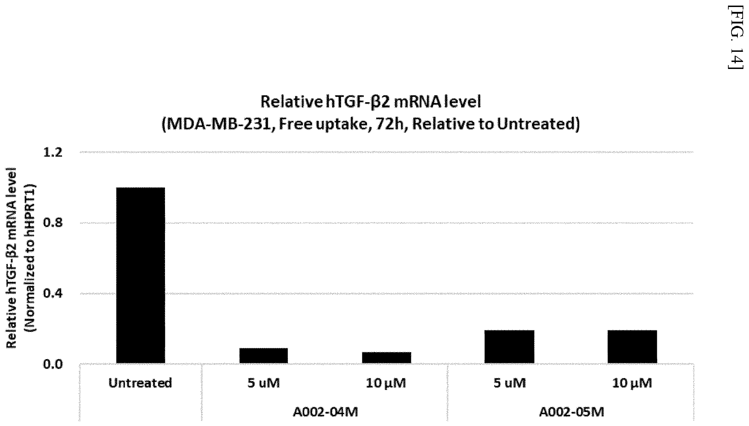

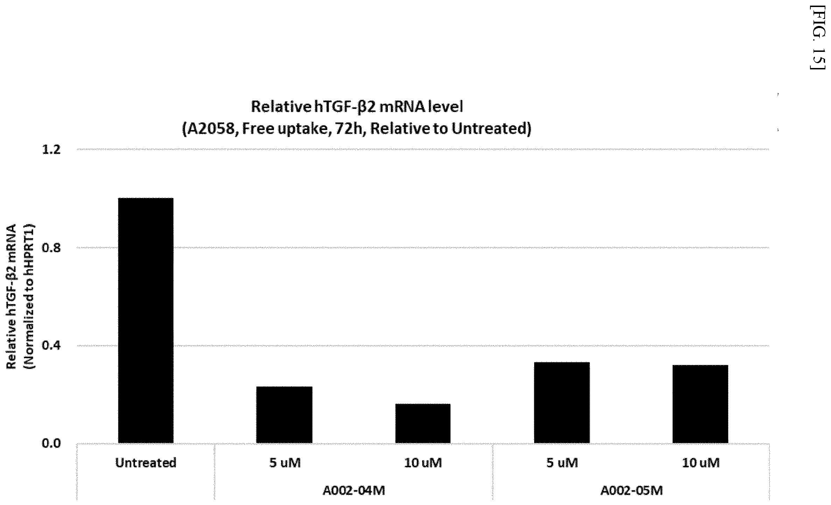

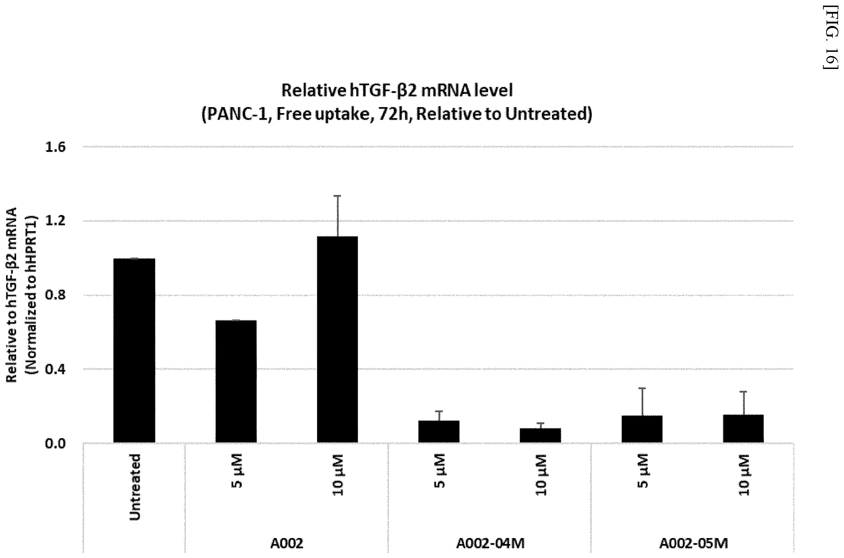

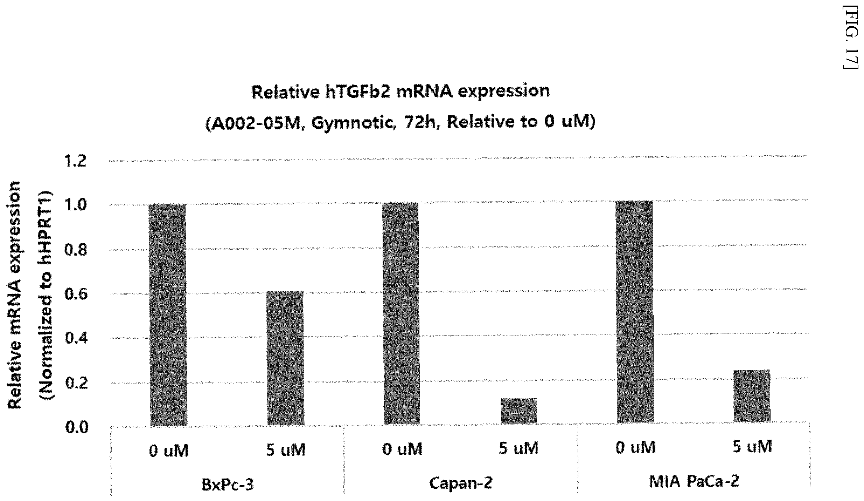

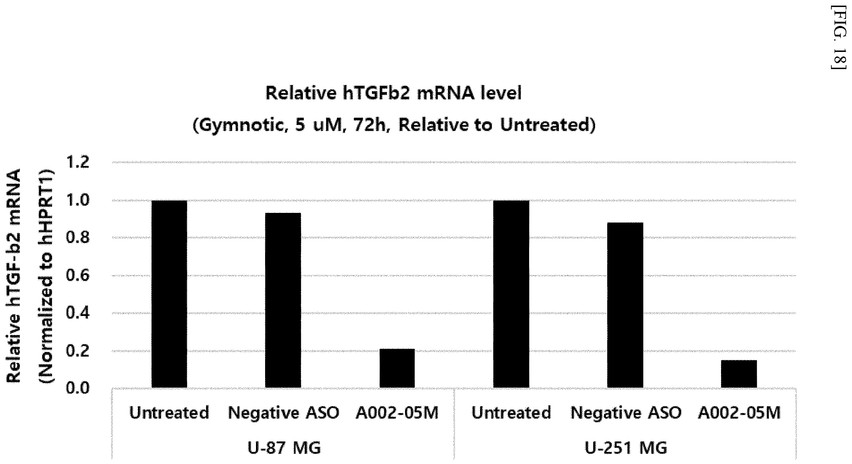

The results of the experiment for each cell line is shown in FIGS. 12 to 18.

As a result of the experiment, it was found that hTGF-β2 mRNA levels were remarkably reduced in various carcinomas (lung cancer, cholangiocarcinoma, triple negative breast cancer, melanoma, pancreatic cancer, and glioblastoma) by A002-04M or A002-05M.

### Experimental Example 12: Evaluation on anticancer efficacy in humanized xenograft mouse models transplanted with melanoma cell line A2058

Humanized A2058 xenograft model:
Mouse: NSG-b2m immunodeficient mice, females 5-7 weeks old, 6 mice/group

After the mice are obtained, the mice were tamed for a week.

Human peripheral blood mononuclear cells (PBMCs: Zenbio, Cat# SER-PBMC-200-F) were transplanted by intravenous (i.v.) administration with 1×10⁷ cells/mouse.

5 days after PBMC transplantation, the A2058 cell line was transplanted by subcutaneous administration with 2×10⁶ cells/mouse.

5 days after transplantation of the A2058 cell line, administration was initiated through intraperitoneal injection of ASO according to the present disclosure:
Treatment: 3 times a week in a dosage of 30 mg/kg and an administration volume of 10 ml/kg (i.e., the concentration of the injection solution is 30 mg/10 ml ASO)
Observation: Measurement of a body weight and a tumor volume twice a week

The overall experimental design for sets A, B, and C below is similar, but separated by each donor of the transplanted PBMCs.

The experimental design of each group for sets A, B, and C is as shown in Tables 10 to 12 below:

**TABLE 10**

| Set A | | | | | | |
|---|---|---|---|---|---|---|
| Group | N | PBMC | Agent | Dose | Route | Injection interval |
| 1 | 6 | - | PBS | - | IP | TIW |
| 2 | 6 | 1×10⁷ | PBS | - | IP | TIW |
| 3 | 6 | 1×10⁷ | A002-04M | 30 mpk | IP | TIW |
| 4 | 6 | 1×10⁷ | A002-05M | 30 mpk | IP | TIW |

| | | | | | | |
|---|---|---|---|---|---|---|
| TIW = three times per week / ip = intraperitoneal | | | | | | |

**TABLE 11**

| Set B | | | | | | |
|---|---|---|---|---|---|---|
| Group | N | PBMC | Agent | Dose | Route | Injection interval |
| 1 | 6 | - | PBS | - | IP | TIW |
| 2 | 6 | 1×10⁷ | PBS | - | IP | TIW |
| 3 | 6 | 1×10⁷ | A002-05M | 30 mpk | IP | TIW |
| 4 | 6 | 1×10⁷ | A002-08M | 30 mpk | IP | TIW |

**TABLE 12**

| Set C | | | | | | |
|---|---|---|---|---|---|---|
| Group | N | PBMC | Agent | Dose | Route | Injection interval |
| 1 | 6 | - | PBS | - | IP | TIW |
| 2 | 6 | 1×10⁷ | PBS | - | IP | TIW |
| 3 | 6 | 1×10⁷ | A002-04M | 30 mpk | IP | TIW |
| 4 | 6 | 1×10⁷ | A002-05M | 30 mpk | IP | TIW |
| 5 | 6 | 1×10⁷ | A002-08M | 30 mpk | IP | TIW |

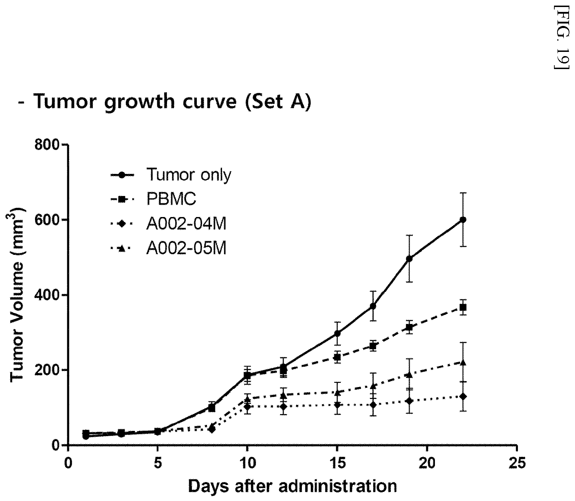

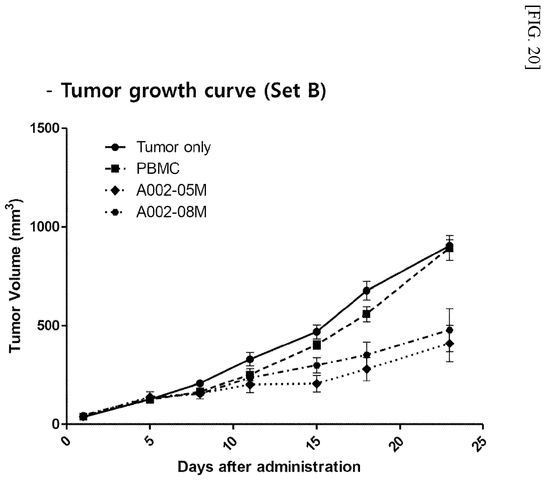

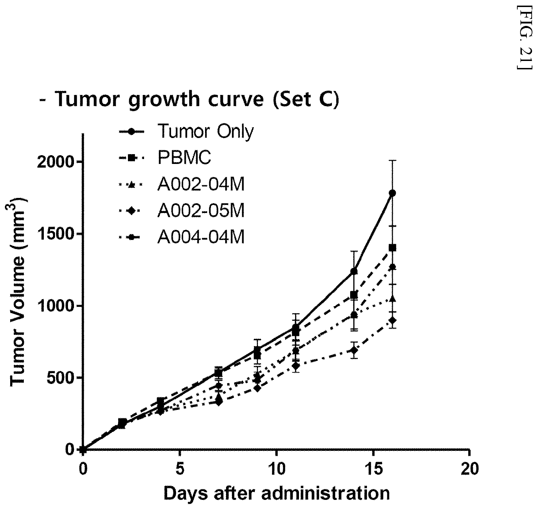

The tumor growth curve for each set is shown in FIG. 19 to FIG. 21.

The tumor growth inhibition rate (IR) for each set is shown in Tables 13 to 15 below:

**TABLE 13**

| - **Tumor growth inhibition rate (IR) (%) (Set A)** | | | | |
|---|---|---|---|---|
| | | G2 (PBMC) | G3 | G4 |
| **Administered substances** | | PBS | A002-04M | A002-05M |
| **Dosage (mg/kg)** | | - | 30 | 30 |
| **Usage** | | - | IP, TIW | IP, TIW |
| Days after admmistra tion | Day 1 | 0.00 | 0.6 | 0 |
| | Day 3 | 0.00 | 4.7 | 4.3 |
| | Day 5 | 0.00 | 2.2 | -0.7 |
| | Day 8 | 0.00 | 57.4 | 47.3 |
| | Day 10 | 0.00 | 44.6** | 32.8* |
| | Day 12 | 0.00 | 47.6** | 31.9* |
| | Day 15 | 0.00 | 53.8** | 39.5** |
| | Day 17 | 0.00 | 58.9** | 39.9* |
| | Day 19 | 0.00 | 62.1** | 39.9* |
| | Day 22 | 0.00 | 64.4** | 39.6* |

**TABLE 14**

| - **Tumor growth inhibition rate (IR) (%) (Set B)** | | | | |
|---|---|---|---|---|
| | | G2 (PBMC) | G3 | G4 |
| **Administered substances** | | PBS | A002-05M | A002-08M |
| **Dosage (mg/kg)** | | - | 30 | 30 |
| **Usage** | | - | IP, TIW | IP, TIW |
| Days after administr ation | Day 1 | 0.0 | -3.8 | -18.9 |
| | Day 5 | 0.0 | -11.2 | -71 |
| | Day 8 | 0.0 | 5.3 | 6.4 |
| | Day 11 | 0.0 | 19.5 | 6.0 |
| | Day 15 | 0.0 | 48.8 * | 25.8 |
| | Day 18 | 0.0 | 50.1 * | 37.1 |
| | Day 23 | 0.0 | 54.2 ** | 46.6 ** |

**TABLE 15**

| - **Tumor growth inhibition rate (IR) (%) (Set C)** | | | | | |
|---|---|---|---|---|---|
| | | G2 (PBMC) | G3 | G4 | G5 |
| **Administered substances** | | PBS | A002-04M | A002-05M | A004-04M |
| **Dosage (mg/kg)** | | - | 30 | 30 | 30 |
| **Usage** | | - | IP, TIW | IP, TIW | IP, TIW |
| Days after adminis tration | Day 2 | 0.0 | 11.2 | 8.2 | 4.9 |
| | Day 4 | 0.0 | 19.6* | 22.1** | 20.8** |
| | Day 7 | 0.0 | 29.5** | 37.8** | 16.4 |
| | Day 9 | 0.0 | 19.6 | 34.6** | 26.5* |
| | Day 11 | 0.0 | 15.4 | 28.3* | 15.1 |
| | Day 14 | 0.0 | 12.6 | 35.6** | 12.4 |
| | Day 16 | 0.0 | 24.9 | 35.8 | 9.2 |

From the result of the experiment, it was determined that the tumor growth inhibition rate upon administration of ASO according to the present disclosure is more superior than the immunotherapeutic effect by PBMC.

### Industrial Applicability

The oligonucleotide of the present disclosure may be used as an anticancer agent by suppressing expression of TGF-β2.

### Sequence List Free Text

SEQ ID NO: 1: 5'-GGCGG CATGT CTATT TTGTA-3'
SEQ ID NO: 2: 5'-CGGCA TGTCT ATTTT GTAAA-3'
SEQ ID NO: 3: 5'-GCGGC ATGTC TATTT TGTAA-3'

## Claims

1. An oligonucleotide which is an antisense oligonucleotide of any one of SEQ ID NOS: 1 to 3,
wherein at least one nucleotide of the oligonucleotide is a nucleotide having a modified nucleoside, and
the modified nucleotide is selected from the group consisting of a 2'-O-methoxyethyl (MOE)-modified nucleotide, a 2'-fluoro (F)-modified nucleotide, a 2'-O-methyl (O-Me)-modified nucleotide, a locked nucleic acid (LNA)-modified nucleotide, an ethylene-bridged nucleic acid (ENA)-modified nucleotide, an (R/S)-constrained ethyl (cET)-modified nucleotide, and a polyalkylene oxide-modified nucleotide.

2. The oligonucleotide of claim 1, wherein one or more nucleotides at a 5'-end or one or more nucleotides at a 3'-end of the oligonucleotide are modified nucleotides.

3. The oligonucleotide of claim 1, wherein one or more nucleotides at a 5'-end and one or more nucleotides at a 3'-end of the oligonucleotide are modified nucleotides.

4. The oligonucleotide of claim 2, wherein the first to n^{th} nucleotides at the 5'- end of the oligonucleotide or the first to m^{th} nucleotides at the 3'-end of the oligonucleotide are the modified nucleotides (here, n and m are each independently an integer of 1 to 10).

5. The oligonucleotide of claim 3, wherein the first to n^{th} nucleotides at the 5'- end of the oligonucleotide and the first to m^{th} nucleotides at the 3'-end of the oligonucleotide are the modified nucleotides.

6. The oligonucleotide of claim 5, wherein the oligonucleotide further comprises one or more modified nucleotides between the (n+1)^{th} nucleotide at the 5'-end and the (m+1)^{th} nucleotide at the 3'-end of the oligonucleotide.

7. An oligonucleotide which is an antisense oligonucleotide represented by any one of SEQ ID NOS: 1 to 30 in Table 1 below,
wherein nucleotides indicated in bold and underlined letters in Table 1 below are modified nucleotides, and
the modified nucleotide is selected from the group consisting of a 2'-O-methoxyethyl (MOE)-modified nucleotide, a 2'-fluoro (F)-modified nucleotide, a 2'-O-methyl (O-Me)-modified nucleotide, a locked nucleic acid (LNA)-modified nucleotide, an ethylene-bridged nucleic acid (ENA)-modified nucleotide, an (R/S)-constrained ethyl (cET)-modified nucleotide, and a polyalkylene oxide-modified nucleotide:

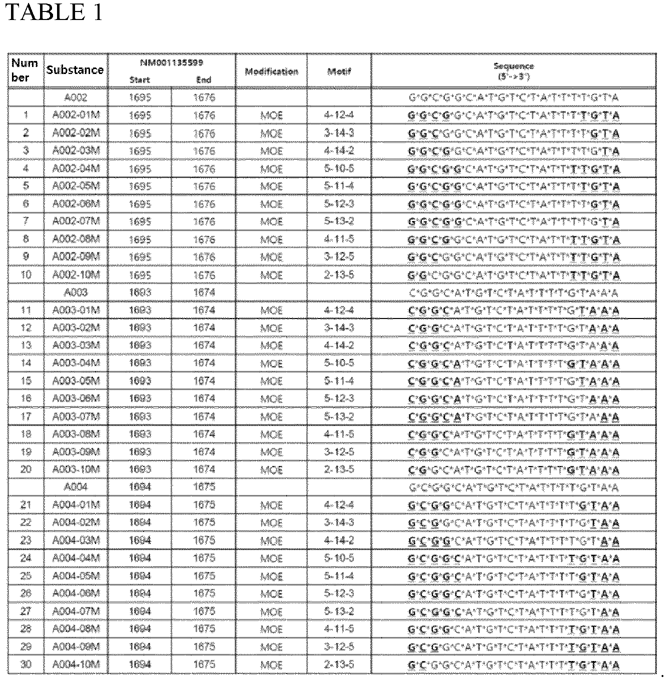

8. The oligonucleotide of claim 7, wherein a modified nucleotide of an oligonucleotide represented by any one of SEQ ID NOS: 1 to 30 in Table 1 is a 2'-O-methoxyethyl (MOE)-modified nucleotide.

9. The oligonucleotide of any one of claims 1 to 8, wherein a bond between neighboring nucleosides is either a phosphodiester bond or a phosphothioate bond.

10. The oligonucleotide of any one of claims 1 to 8, wherein a bond between neighboring nucleosides is a phosphothioate bond.

11. A pharmaceutical composition for treating cancer, comprising the oligonucleotide according to claim 9.
